(19) Europäisches Patentamt
European Patent Office
Office européen des brevets

(11) Publication number: **0 090 378**
**B1**

(12) **EUROPEAN PATENT SPECIFICATION**

(45) Date of publication of patent specification: **23.07.86**

(51) Int. Cl.$^4$: **C 07 C 57/03**, C 07 C 51/42, A 61 K 31/20

(21) Application number: **83102998.8**

(22) Date of filing: **25.03.83**

(54) **Isoprenylcarboxylic acid-containing composition for external use.**

(30) Priority: **26.03.82 JP 47287/82**

(43) Date of publication of application:
**05.10.83 Bulletin 83/40**

(45) Publication of the grant of the patent:
**23.07.86 Bulletin 86/30**

(84) Designated Contracting States:
**BE CH DE FR GB IT LI NL SE**

(58) References cited:
**EP-A-0 054 732**
**DE-A-2 934 090**
**GB-A-1 476 717**
**GB-A-1 580 444**
**GB-A-2 073 750**

**HAGERS HANDBUCH der pharmazeutischen Praxis, Vol. 7, Part A (1971), 559**

(73) Proprietor: **Eisai Co., Ltd.**
**6-10, Koishikawa 4-chome Bunkyo-ku**
**Tokyo 112 (JP)**

(72) Inventor: **Deushi, Kouhei**
**380, Kodama, Kodamamachi**
**Kodama-gun Saitama (JP)**
Inventor: **Ohsawa, Shigemitsu**
**2286-12, Suehiro-cho**
**Honjo-shi Saitama (JP)**
Inventor: **Kaneko, Genichiro**
**1315, Serada Ojimamachi**
**Nitta-gun Gunma (JP)**
Inventor: **Amada, Iwao**
**644-1, Naka Fujioka-shi**
**Gunma (JP)**

(74) Representative: **Hansen, Bernd, Dr.rer.nat. et al**
**Hoffmann, Eitle & Partner Patentanwälte**
**Arabellastrasse 4**
**D-8000 München 81 (DE)**

**EP 0 090 378 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to an isoprenylcarboxylic acid-containing composition for external (topical) use.

The isoprenylcarboxylic acid employed in the composition according to the present invention is all-trans-3,7,11,15-tetramethyl-2,4,6,10,14-hexadecapentaenoic acid-1 of the following formula:

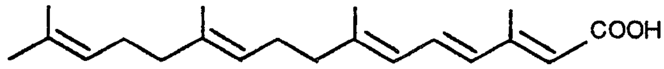

The medical use of this compound as a carcinostatic substance or for treatment of psoriasis has been described in GB—A—2 073 750. However, it is evident from its chemical structure that this compound is easily oxidizable. In fact, it is easily oxidized and its titer is reduced, as is shown in the experimental examples given below (see control samples).

Particularly when the foregoing compound is incorporated in a composition to be used as a topical drug for treatment of psoriasis, it is likely to undergo oxidation and, therefore, it becomes quite dificult to attain the intended medicinal effects.

In Ullmanns Encyclopaedia of Technical Chemistry, 4th edition, volume 12 (1976), page 561, right column, and volume 8 (1974), pages 25 and 41, it is described to add antioxidants to pharmaceutical or cosmetical compositions containing unmsaturate fatty acids.

After investigations made under these circumstances for the purpose of finding antioxidants that can be incorporated in a composition for external use containing an isoprenylcarboxylic acid, which antioxidants are capable of preventing or retarding oxidation of an isoprenylcarboxylic acid, the inventors have found that antioxidants specified according to the present invention are capable of attaining such effects. The present invention has been completed on the basis of this finding.

An object of the present invention is, therefore, to provide a composition for external pharmaceutical use containing an isoprenylcarboxylic acid according to the invention in which the acid can be maintained in a stable state.

Fig. 1 is a graph showing the changes in residual amount of an isoprenylcarboxylic acid with the passing of time, as obtained in Comparative Example.

Fig. 2 is a graph showing the changes in residual amount of an isoprenylcarboxylic acid, according to the present invention, with the passing of time, as obtained in Example 1.

The present invention will now be described.

The composition of the present invention is to be applied externally to the skin. As a base for the inventive composition, an emulsion-type base of any conventional type useful for the preparation of medicated ointments, creams, lotions and gels is used.

For said emulsion-type base, the isoprenylcarboxylic acid according to the present invention is dissolved in both the oily and aqueous phases to form a composition for external use such as a cream lotion or gel.

As base for the oily phase can be used white petrolatum, sesame oil, liquid paraffin, cetyl alcohol, oleyl alcohol, oleic acid or isopropylmyristate. For the aqueous phase it is preferable that this is an aqueous solution of a surfactant, such as polyethylene-hardened castor oil, or an aqueous solution containing a surfactant mixed with a solubilizing solvent, such as propylene glycol.

The amount of antioxidant(s) varies depending on the amount of isoprenylcarboxylic acid used according to the present invention. Generally, the amount of said isoprenylcarboxylic acid in the composition for external use in 0.1 to 1.0 wt.%, although the present invention is not limited to this range. The total amount of the antioxidant(s) should be an amount in the range of 0.005 to 0.5 wt.%, preferably 0.01 to 0.3 wt.%, in order to obtain the effects of the present invention when the foregoing amount of the isoprenylcarboxylic acid is used. However, the amount of the antioxidant or antioxidants is not limited to this range, in the present invention.

The composition of the present invention can be prepared as follows.

For the emulsion-type base, the surfactant, the oily or fatty base and a first antioxidant are charged into a vessel while the vessel is being continuously purged with $N_2$ gas, and the mixture is heated to obtain a homogenous mixture. The isoprenylcarboxylic acid according to the present invention is than added thereto and the resulting mixture is stirred. A second antioxidant and desirably an aqueous solution of EDTA are then added thereto to produce an emulsion. The obtained emulsion is immediately cooled and the vessel is again purged with $N_2$ gas.

The effects of the present invention will be illustrated by the following examples. In the examples, the term "percent" means percent by weight. The term "residual amount (%)" means the

$$\frac{\text{measured amount of the isoprenylcarboxylic acid}}{\text{starting amount of the isoprenylcarboxylic acid}} \times 100$$

Comparative Example

Four types of samples were prepared using oleic acid, oleyl alcohol, isopropyl myristate and sesame oil, respectively, as the oily or fatty bases. The compound 3,7,11,15-tetramethyl-2,4,6,10,14-hexadecapentaenoic acid-1, hereinafter referred to as ACT, was added separately to each base to obtain 0.4 w/w % solutions to be used as samples. The samples were charged into ampoules and were heated to 70°C, for various times. The residual amounts (%) of the starting isoprenylcarboxylic acid in the ampoules were measured at various times. The determination was effected by diluting the samples with chloroform and determining the UV absorption at 321 nm.

The results of the above comparative example are shown in Fig. 1. In Fig. 1, the lines with the symbols ⊗, ◎, ◬, and ○ show the changes in the residual amounts obtained when oleic acid, oleyl alcohol, isopropyl myristate and sesame oil were used, respectively.

Example 1

Samples and Methods:

Samples K, O and P were prepared according to the recipes shown in Table 1. These sampes were prepared by heating sesame oil, oleyl alcohol and polyoxyethylene (60 moles) hydrogenated castor oil to 85°C, and then adding, if called for butylated hydroxytoluene (BHT) or ascorbic acid monostearate thereto, stirring the mixture for 5 min, adding thereto isoprenylcarboxylic acid and stirring the mixture again for 10 min. Then an aqueous solution heated to 90°C, which was prepared previously by dissolving EDTA and, for some samples, ascorbic acid, in water, was added, and the resulting mixture was cooled and emulsified immediately thereafter. Then, the samples were heated to 70°C and the residual amounts of the isoprenylcarboxylic acid, with the passing of time, were determined by means of HPLC.

TABLE 1

| Starting Material | Sample K (comparative) | O | P |
|---|---|---|---|
| ACT | 0.2 % | 0.2 % | 0.2 % |
| sesame oil | 1.0 | 1.0 | 1.0 |
| oleyl alcohol | 1.0 | 1.0 | 1.0 |
| polyoxyethylene hydro-generated castor oil | 2.0 | 2.0 | 2.0 |
| BHT | — | 0.05 | — |
| ascorbic acid monostearate | — | — | 0.05 |
| Ascorbic acid | — | 0.1 | 0.1 |
| EDTA-2Na | — | 0.05 | 0.05 |
| Water | balance to 100% | balance to 100% | balance to 100% |

BHT: Butylated hydroxytoluene

Results:

The results are shown in Figure 2. In Figure 2, the lines connecting symbols ⊙, ⦶ and ⊖, respectively, show the changes in the residual amounts of samples K, O and P, respectively, over time. The effects of the present invention are evident from Fig. 2.

Example 2:

0.2 % of isoprenylcarboxylic acid, hereinafter referred to as ACT, 3 % of white petrolatum, 12 % of liquid paraffin, 10 % of stearyl alcohol, 2 % of polyoxyethylene monocetyl ether (Cetomacrogol ®) and 0.05 % of butylated hydroxytoluene were heated to 75°C to obtain a melt which will be referred to as component A. Separately, 0.025 % of methylparaben, 0.015 % of propylparaaben, 5.0 % of propylene glycol, 0.04 % of ascorbic acid and 0.05 % of EDTA-4Na were dissolved in water (to make 100 %). The solution was heated to 75°C to obtain component B. Component B was added slowly to component A under stirring in an $N_2$ gas stream to obtain an emulsion. The emulsion was treated in a conventional manner to obtain a hydrophilic cream. In this example, percentages are by weight based on the total weight of the hydrophilic cream.

A control, the same hydrophilic cream as described above but not containing butylated hydroxytoluene and ascorbic acid, was also prepared. The product of this example and the control were stored at 45°C for 3 months and the residual amounts of ACT were examined. The obtained values for the control and the product of this example were 82.4 % and 97.7 %, respectively.

Example 3

0.2 % of ACT, 0.4 % of fatty acid triglyceride (Miglyol ®), 4.0 % of polyoxyethylene-hardened castor oil (60) and 0.05 % of ascorbic acid monostearate were heated to 80°C to obtain a melt which will be referred to as component A. Separately, 0.05 % of erythorbic acid, 0.05 wt.% of EDTA-2Na, 0.1 % of methylparaben and 10.0 % of propylene glycol were dissolved in water (to make 100 %). The solution was heated to 80°C to obtain component B. Component B was added to component A under stirring in $N_2$ gas stream to prepare a lotion in a conventional manner. The percentages for this weight, based on the total weight of the lotion.

A control, the same lotion as described above but not containing ascorbic acid monostearate and erythorbic acid, was also prepared. The product of this example and the control were stored at 45°C for 3 months and the residual amounts of ACT were examined. The obtained values for the control and the product of this example were 78.4 % and 95.7 %, respectively.

Example 4

0.2 % of ACT and 0.05 % of ascorbic acid monopalmitate were dissolved in 0.6 % of isopropyl myristate, 0.5 % of polyoxyethylene oleyl alcohol ether and 25.0 % of isopropyl alcohol to obtain component A. Separately, 0.04 % of phytic acid and 0.05 % of ascorbic acid were dissolved in 5.0 % of propylene glycol and water (to make 100 %). 1.0 % of carboxyvinyl polymer (Carbopol 34 ®) was added to the solution to obtain a homogeneous dispersion which will be referred to as component B. Component A was added to component B. The mixture was adjusted to pH 6.5 by adding sodium hydroxide under stirring. The mixture was stirred in an $N_2$ gas stream to obtain a gel. In this example, percentages are by weight based on the total weight of the gel.

A control, the same gel as described above but not containing ascorbic acid monopalmitate and ascorbic acid, was also prepared. The product of this example and the control were stored at 40°C for 3 months and the residual amounts of ACT in the control and the product of this example were measured which showed that the residual amounts were 66.5 % and 93.8 %, respectively.

Example 5

0.2 % of ACT, 0.05 % of butylated hydroxyanisole, 24 % of stearyl alcohol, 24 % of white petrolatum and 5 % of polyoxyethylene monostearate were heated to 80°C to obtain a melt which will be referred to as component A. Separately, 12 % of propylen glycol and 0.05 % of sodium hydrogensulfite were dissolved in water (to make 100 %). The solution was heated to 80°C to obtain component B. Component B was added to component A under stirring in an $N_2$ gas stream to obtain a hydrophilic ointmemnt in a conventional manner. In this example, percentages are by weight based on the total weight of hydrophilic ointment.

A control, the same hydrophilic ointment as described above but not containing butylated hydroxyanisole and sodium hydrogensulfite, was also prepared. The product of this example and the control were stored at 45°C for 3 months and the residual amounts of ACT in the control and the product of this example were measured which showed that these residual amounts were 85.6 % and 98.5 %, respectively.

Example 6

0.2 % of ACT, 1 % of stearyl alcohol, 30 % of liquid paraffin, 4 % of sorbitan monostearate, 0.03 % of butylated hydroxytoluene and 0.02 % of propyl gallate were heated to 80°C to obtain a melt, which will be referred to as component A. Separately, 4 % of polyoxyethylene sorbitan monostearate, 0.05 % of EDTA-2Na, 0.15 % of hydroxyethylcellulose and 0.05 % of cysteine hydrochloride were dissolved in water (to make 100 %). The solution was heated to 80°C to obtain component B. Component B was added to component A under stirring in an $N_2$ gas stream to obtain a lotion in a conventional manner. In this example, percentages are by weight based on the total weight of the lotion. A control, the same lotion as described above but not containing butylated hydroxytoluene, propyl gallate and cysteine hydrochloride, was also prepared. The product of this example and the control were stored at 45°C for 3 months and the residual amounts of ACT in the control and the product of this example were measured which showed that these residual amounts were 72.0 % and 95.0 %, respectively.

**Claims**

1. A pharmaceutical composition for external use for the treatment of skin conditions comprising a therapeutically effective amount of a compound having the formula

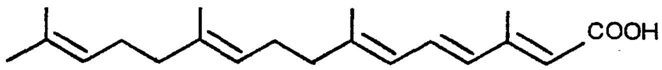

in an emulsion base, characterised in that it comprises an aqueous phase and an oily or fatty phase, wherein said oily of fatty phase contains an antioxidant selected from the group consisting of butylated hydroxytoluene, butulated hydroxyanisole, fatty acid esters of ascorbic acid, tocopherol and gallic acid esters, and said aqueous phase contains an antioxidant selected from the group consisting of ascorbic acid, sodium ascorbate, erythorbic acid, sodium erythorbate, cysteine, sodium hydrogensulfite and sodium metabisulfite.

2. The pharmaceutical composition according to claim 1, wherein said antioxidant is contained in said composition in an amount of 0.005 to 0.5 wt.%, based on the total weight of said composition.

3. The pharmaceutical composition according to claim 1, wherein the amount of said antioxidant is in the range of 0.01 to 0.3 wt.%, based on the total weight of said composition.

4. The pharmaceutical composition according to one of the claims 1 to 3, wherein the amount of isoprenylcarboxylic acid is in the range of 0.1 to 1.0 wt.%, based on the total weight of said composition.

5. The pharmaceutical composition according to claim 1, wherein said oily base or fatty base is selected from the group consisting of white petrolatum, sesame oil, liquid paraffin, cetyl alcohol, oleyl alcohol, oleic acid and isopropylmyristate.

6. The pharmaceutical composition according to claim 1, wherein said aqueous base is an aqueous solution of a surfactant.

7. The pharmaceutical composition according to claim 1 and 6, wherein said surfactant is polyoxyethylene-hardened castor oil.

8. The pharmaceutical composition according to claim 7, wherein said aqueous solution further contains a solvent for solubilizing said compound.

9. The pharmaceutical composition according to claim 8, wherein said aqueous solution further contains an effective amount of propylene glycol as a solvent for solubilizing said compound.

10. The pharmaceutical composition according to claim 9, prepared by dissolving isoprenylcarboxylic acid in a small quantity of sesame oil and adding the resulting solution to said aqueous solution.

11. The pharmaceutical composition according to claim 1, wherein said emulsion further contains an effective amount of EDTA.

12. A pharmaceutical composition adapted for external use for the treatment of skin diseases, comprising 0.1 to 1.0 wt.% of a compound of the formula

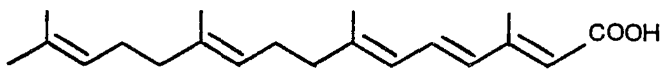

characterised in that is comprises in an emulsion phase consisting essentially of an aqueous phase and an oily of fatty phase, wherein said oily and fatty phase contains 0.005 to 0.5 wt.% antioxidant selected from the group consisiting of butylated hydroxytoluene, butylated hydroxyanisole, fatty acid esters of ascorbic acid, tocopherol and gallic acid esters, and said aqueous phase contains 0.005 to 0.5 wt.% antioxidant selected from the group consisting of ascorbic acid, sodium ascorbate, erythorbic acid, sodium erythorbate, cysteine, sodium hydrogensulfite and sodium metabisulfite.

**Patentansprüche**

1. Pharmazeutische Zusammensetzung zur externen Anwendung bei der Behandlung von Hautproblemen, welche eine therapeutisch wirksame Menge einer Verbindung der folgenden Formel

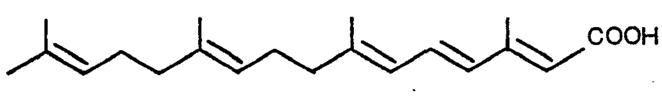

in einer Emulsion umfaßt, dadurch gekennzeichnet, daß sie eine wäßrige und eine öl- oder Fettphase umfaßt, worin die genannte öl- oder Fettphase ein Antioxydans enthält, welches ausgewählt ist aus der Gruppe bestehend aus butyliertem Hydroxytoluol, butyliertem Hydroxyanisol, Fettsäureestern von Asorbinsäure, Tocopherol und Gallussäureester, und die genannte wäßrige Phase ein Antioxydans enthält, welches ausgewählt ist aus der Gruppe bestehend aus Ascorbinsäure, Natriumascorbat, Isoascorbinsäure, Natriumisoascorbat, Cystein, Natriumhydrogensulfit und Natriummetabisulfit.

2. Pharmazeutische Zusammensetzung nach Anspruch 1, dadurch gekennzeichnet, daß das Antioxydans in der genannten Zusammensetung in einer Menge von 0,005 bis 0,5 Gew.%, bezogen auf das Gesamtgewicht der Zusammensetzung, enthalten ist.

3. Pharmazeutische Zusammensetzung nach Anspruch 1, dadurch gekennzeichnet, daß die Menge des genannten Antioxydans im Bereich von 0,01 bis 0,3 Gew.%, bezogen auf das Gesamtgewicht der genannten Zusammensetzung, liegt.

4. Pharmazeutische Zusammensetzung nach einem der Ansprüche 1 bis 3, dadurch gekennzeichnet, daß die Menge an Isoprenylcarbonsäure im Bereich von 0,1 bis 1,0 Gew.%, bezogen auf das Gesamtgewicht der genannten Zusammensetzung, liegt.

5. Pharmazeutische Zusammensetzung nach Anspruch 1, dadurch gekennzeichnet, daß die genannte ölbasis oder Fettbasisausgewählt ist aus der Gruppe bestehend aus Weißöl, Sesamöl, flüssigem Paraffin, Cetylalkohol, Oleylalkohol, Oleinsäure und Isopropylmyristat.

6. Pharmazeutische Zusammensetzung nach Anspruch 1, dadurch gekennzeichnet, daß die wäßrige Basis eine wäßrige Lösung eines oberflächenaktiven Mittels darstellt.

7. Pharmazeutische Zusammensetzung nach Anspruch 1 und 6, dadurch gekennzeichnet, daß das genannte oberflächenaktive Mittel Polyoxyethylen-gehärtetes Rizinusöl darstellt.

8. Pharmazeutische Zusammensetzung nach Anspruch 7, dadurch gekennzeichnet, daß die wäßrige Lösung im weiteren ein Lösungsmittel zum Solubilisieren der genannten Verbindung enthält.

9. Pharmazeutische Zusammensetzung nach Anspruch 8, dadurch gekennzeichnet, daß die genannte wäßrige Lösung im weiteren eine wirksame Menge an Propylenglykol als Lösungsmittel zum Solubilisieren der genannten Verbindung enthält.

10. Pharmazeutische Zusammensetzung nach Anspruch 9, hergestellt durch Auflösen von Isoprenylcarbonsäure in einer kleinen Menge Sesamöl und Zugabe der erhaltenen Lösung zu der genannten wäßrigen Lösung.

11. Pharmazeutische Zusammensetzung nach Anspruch 1, dadurch gekennzeichnet, daß die genannte Emulsion im weiteren eine wirksame Menge an EDTA enthält.

12. Pharmazeutische Zusammensetzung zur externen Anwendung bei der Behandlung von Hauterkrankungen, welche 0,1 bis 1,0 Gew.% einer Verbindung der folgenden Formel

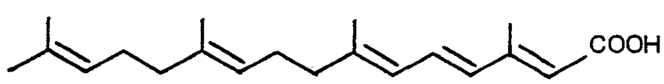

umfaßt, gekennzeichnet durch eine Emulsionsphase, welche im wesentlichen aus einer wäßrigen Phase und einer öl- oder Fettphase besteht, wobei die genannte öl- und Fettphase 0,005 bis 0,5 Gew.% Antioxydans, ausgewählt aus der Gruppe bestehend aus butyliertem Hydroxytoluol, butyliertem Hydroxyanisol, Fettsäureestern der Ascorbinsäure, Tocopherol und Gallussäureestern enthält, und die genannte wäßrige Phase 0,005 bis 0,5 Gew.% Antioxydans, ausgewählt aus der Gruppe bestehend aus Ascorbinsäure, Natriumascorbat, Isoascorbinsäure, Natriumisoascorbat, Cystein, Natriumhydrogensulfit und Natriummetabisulfit enthält.

**Revendications**

1. Composition pharmaceutique à usage externe pour le traitement de maladies de la peau comprenant une quantité thérapeutiquement active d'un composé ayant la formule:

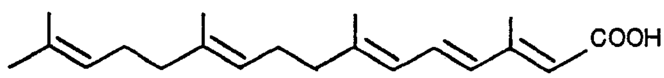

dans une base d'emulsion, caractérisée en ce qu'elle comprend une phase aqueuse et une phase huileuse ou grasse, la phase huileuse ou grasse contenant un antioxydant choisi parmi l'hydroxytoluènebutylé, l'hydroxyanisolebutylé, les esters d'acide gras de l'acide ascorbique, le tocophérol et les esters de l'acide gallique, et la phase aqueuse contenant un anti-oxydant choisi parmi l'acide ascorbique, l'ascorbate de sodium, l'acide érythorbique, l'érythorbate de sodium, la cystéine, l'hydrogénsulfite de sodium et le métabisulfite de sodium.

2. Composition pharmaceutique selon la revendication 1, caractérisée en ce que l'anti-oxydant est contenu dans la composition en quantité de 0,005 à 0,5 % en poids, par rapport au poids total de la composition.

3. Composition pharmaceutique selon la revendication 1, caractérisée en ce que la teneur en anti-oxydant est dans l'intervalle de 0,01 à 0,3 % en poids par rapport au poids total de la composition.

4. Composition pharmaceutique selon l'une quelconque des revendications 1 à 3, caractérisée en ce que la teneur en acide isoprénylcarboxylique est dans l'intervalle de 0,1 à 1,0 % en poids par rapport au poids total de la composition.

5. Composition pharmaceutique selon la revendication 1, caractérisée en ce que la base huileuse ou base grasse est choisie parmi la vaseline blanche, l'huile de sésame, la paraffine liquide, l'alcool cétylique, l'alcool oléylique, l'acide oléique et le myristate d'isopropyle.

6. Composition pharmaceutique selon la revendication 1, caractérisée en ce que la base aqueuse est une solution aqueuse d'un tensio-actif.

7. Composition pharmaceutique selon les revendications 1 et 6, , caractérisée en ce que le tensio-actif est l'huile de ricin durcie polyoxyéthylénée.

8. Composition pharmaceutique selon la revendication 7, caractérisée en ce que la solution aqueuse contient en outre un solvant pour solubiliser le composé.

9. Composition pharmaceutique selon la revendication 8, caractérisée en ce que la solution aqueuse contient en outre une quantité efficace de propylèneglycol comme solvant pour solubiliser le composé.

10. Composition pharmaceutique selon la revendication 9, préparée par dissolution d'acide isoprénylcarboxylique dans une petite quantité d'huile de sésame et addition de la solution obtenue à la solution aqueuse.

11. Composition pharmaceutique selon la revendication 1, caractérisée en ce que l'émulsion contient en outre un quantité efficace d'EDTA.

12. Composition pharmaceutique appropriée à l'usage externe pour le traitement de maladies de la peau, comprenant de 0,1 à 1,0 % en poids d'un composé de formule:

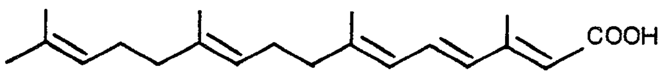

caractérisée en ce qu'elle comprend une phase d'émulsion constituée essentiellement d'une phase aqueuse et d'une phase huileuse ou grasse, la phase huileuse et grasse contenant de 0,005 à 0,5 % en poids d'anti-oxydant choisi parmi l'hydroxytoluènebutylé, l'hydroxyanisolebutylé, les esters d'acide grad de l'acide ascobique, le tocophérol et les esters d'acide gallique, et la phase aqueuse contenant de 0,005 à 0,5 % en poids d'un anti-oxydant choisi parmi l'acide ascorbique, l'ascorbate de sodium, l'acide érythorbique, l'érythorbate de sodium, la cystéine, l'hydrogéno sulfite de sodium et le métabisulfite de sodium.

FIG. 1

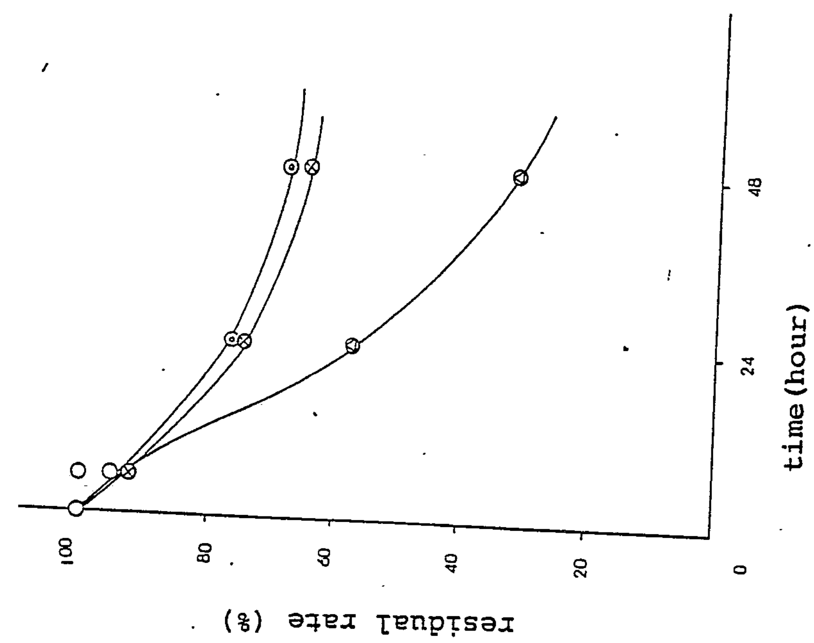
residual rate (%)
100
80
60
40
20
0
24
48
time (hour)

FIG. 2

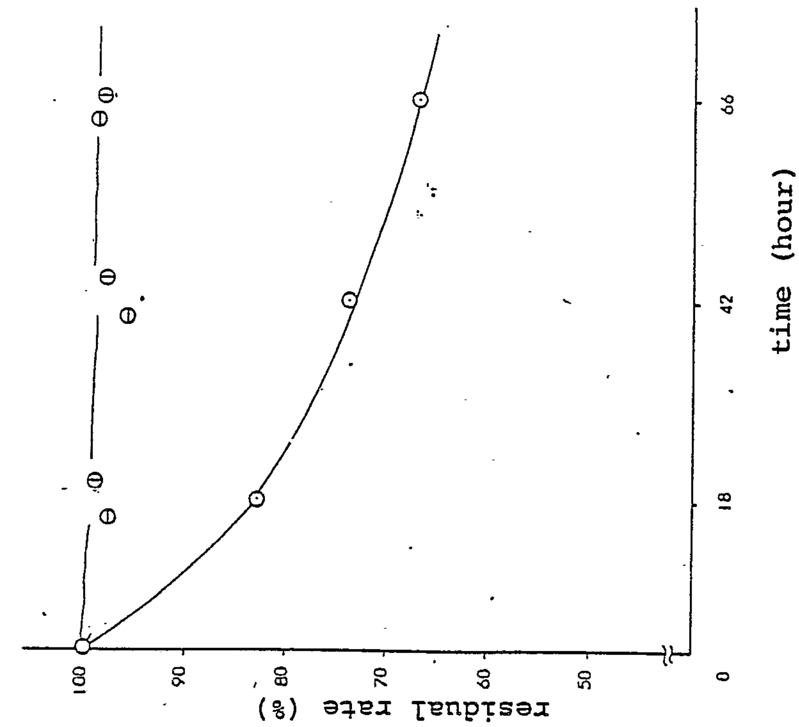
residual rate (%)
100
90
80
70
60
50
0
18
42
66
time (hour)